# EUROPEAN PATENT APPLICATION

(11) **EP 2 551 269 A1**
(43) Date of publication of application: **30.01.2013**
(21) Application number: 12178058.9
(22) Date of filing: 26.07.2012
(51) Int. Cl.: C07D 471/08

(54) **Process for the preparation of varenicline**

(30) Priority: 28.07.2011 IT MI20111413
(71) Applicant: Dipharma Francis S.r.l., 20021 Baranzate (MI) (IT)
(72) Inventor: Attolino, Emanuele, 20021 BARANZATE (MI) (IT); Rossi,Roberto, 20021 BARANZATE (MI) (IT); Allegrini, Pietro, 20021 BARANZATE (MI) (IT)
(74) Representative: Minoja, Fabrizio

(57) **Abstract**

It is disclosed a process for the preparation of a compound of formula **(I)** or a salt thereof, comprising: 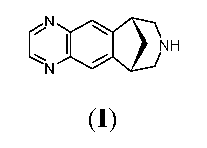 nitrating a compound of formula **(II)** or a salt thereof, 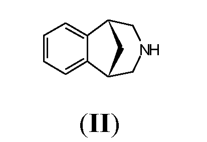 to obtain a compound of formula **(IV)** or a salt thereof, 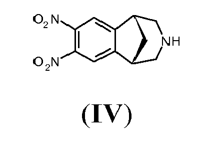 reducing it, to obtain a compound of formula **(V)** or a salt thereof, and 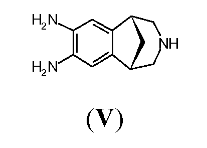 subsequently cyclizing it to obtain a compound of formula **(I)** or a salt thereof and, if desired, converting a compound of formula **(I)** to a salt thereof, or vice versa, characterized in that: the nitration of a compound of formula **(II)** or a salt thereof is carried out with concentrated nitric acid in the presence of a strong inorganic acid and that the amino group in the compound of formula **(II)** is not protected.

## Description

### FIELD OF THE INVENTION

The present invention relates to a process for the preparation of 7,8,9,10-tetrahydro-6,10-methane-6*H*-azepino-[4,5-*g*]-quinoxaline and salts thereof and intermediates useful in its preparation.

### BACKGROUND ART

Varenicline, (7,8,9,10-tetrahydro-6,10-methane-6*H*-azepino-[4,5-*g*]-quinoxaline) of formula **(I)** is a known selective inhibitor of the nicotinic receptors, used as tartrate salt in the treatment of smoke-addition.

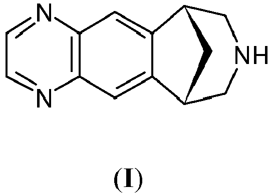

US 6,410,550 discloses its preparation with a total yield of 30% starting from the intermediate of formula (II). 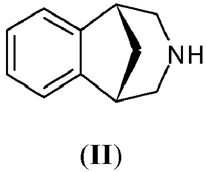 The process comprises: 1) protecting the amine group as trifluoroacetamide, 2) nitrating the aromatic ring in trifluoromethanesulfonic acid, 3) reducing the nitro groups to amine groups, 4) condensing the diamine with the glyoxal bisulphite adduct and 5) deprotecting the amino group. Even if the yields of each single step are acceptable, this preparation suffers from the use of an amino protecting group, necessary to protect the amino group during the nitration reaction which is carried out using an excess of expensive trifluoromethanesulphonic acid.

As it can be noticed, this process is not so efficient, very expensive and difficult to be used on industrial scale.

Furthermore, the amine of formula **(II)** used as intermediate is difficult to be obtained on industrial scale.

Even if various possible synthetic routes are reported in literature (see for example Journal of Organic Chemistry 2004, 69, 5756; Synthesis 2004, 1755; e Organic Letters 2004, 6, 2357), the most interesting one, in terms of process efficiency, is disclosed in US 6,410,550. Anyway, the reagents involved this synthetic route are difficult to be used on industrial scale.

According to this synthetic route, a compound of formula **(III)** is converted into a compound of formula **(II),** or a salt thereof, 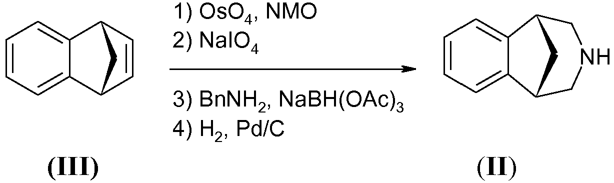 (wherein NMO is N-methylmorpholine-N-oxide)
by a process comprising four reactions, wherein the *cis*-dihydroxylation of benzonorbornadiene of formula **(III)** is carried out with osmium tetroxide, As known, osmium is a heavy, toxic and very expensive metal, which has to be removed from the product and then disposed of the toxic production effluent wastes.

There is therefore the need for a new synthetic route to be applied on industrial scale which provides Varenicline or a salt thereof, and the key intermediate of formula **(II)** or a salt thereof, from low cost starting materials. Besides, the process should be efficient in terms of yield and purity of the obtained product and, most of all, advantageous on industrial scale.

### SUMMARY OF THE INVENTION

It has been surprisingly found a process for the preparation of Varenicline or a salt thereof starting from a compound of formula **(II)** characterized by few steps and without the use of protecting groups.

Furthermore, the invention provides a method for preparing the compound **(II)** which implies the use of an osmium compound supported on a resin, which is safer, recoverable at the end of the reaction and reusable in the same facility. This renders the process of the invention more advantageous on industrial scale compared to the known methods.

### DETAILED DESCRIPTION OF THE INVENTION

The object of this invention is a process for the preparation of a compound of formula **(I)** or a salt thereof, comprising: 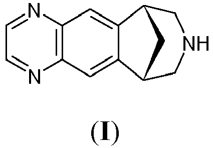 nitrating a compound of formula **(II)** or a salt thereof, 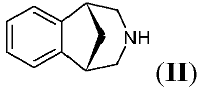 to obtain a compound of formula **(IV)** or a salt thereof, 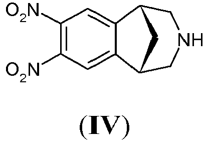 reducing it, to obtain a compound of formula **(V)** or a salt thereof, and then 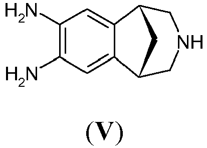 cyclizing it, to obtain a compound of formula **(I)** or a salt thereof, and, if desired, converting a compound of formula **(I)** to a salt thereof, or the salt of a compound of formula **(I)** to the free compound **characterized in that**:
the nitration of a compound of formula **(II),** or a salt thereof, is carried out with concentrated nitric acid in the presence of a strong inorganic acid, and the amino group in the compound of formula **(II)** is not protected.

The concentrated nitric acid is preferably fuming nitric acid.

A strong inorganic acid is chosen for example from sulphuric acid, polyphosphoric acid, and perchloric acid, preferably sulphuric acid.

The same strong inorganic acid can be the reaction solvent; and, if necessary, the reaction can be carried out in the presence of an excess of the acid.

The temperature of the reaction can be comprised between about -10°C and about 60°C, preferably between about 0°C and about 50°C.

The reduction of a compound of formula **(IV),** or a salt thereof, as defined above to obtain a compound of formula **(V)** or a salt thereof, as defined above, and subsequent cyclization to obtain a compound of formula **(I)** or a salt thereof can be carried out according to known methods for example as disclosed in US 6,410,550.

The reduction of a compound of formula **(IV)** or a salt thereof can be carried out by hydrogenation or by treatment with a metal reducing agent in the presence of a strong protic acid.

The hydrogenation, and typically the catalytic hydrogenation can be carried out for example with hydrogen and a metal, homogeneous or heterogeneous catalyst, for example based on palladium, platinum, nickel, rhodium or ruthenium, or derivatives thereof for example salts or complexes thereof.

The catalytic hydrogenation reaction can be carried out using hydrogen at a pressure which can range between about 1 atm and 40 atm. If the metal catalyst is a heterogeneous one, it is preferably deposited on an inert support such as, for example, carbon, barium hydroxide, alumina, calcium carbonate, preferably carbon.

The concentration of the metal on the support can range between about 1% and 30%, preferably between about 2% and 7%.

If desired, the reaction can be carried out in the presence of a solvent selected among a polar aprotic solvent, typically dimethylformamide, dimethylacetamide, acetonitrile, dimethylsulphoxide; a cyclic or acyclic ether, typically tetrahydrofuran or dioxane or methyl tert-butyl ether; a straight or branched C₁-C₆ alkanol, for example methanol, ethanol, isopropanol; a C₁-C₅ carboxylic acid for example acetic or propionic acid, or a mixture of two or more, preferably two or three, of said solvents; or in water or in an aqueous solution of an organic or inorganic protic acid such as, for example, trifluoroacetic acid, methanesulphonic acid, hydrochloric acid or a mixture of two or more of said solvents.

A metal reducing agent can be for example metal zinc, tin or metal iron in acetic acid or in an aqueous solution of a inorganic acid, preferably hydrochloric acid, a salt of tin (II) in an aqueous solution of a inorganic acid, preferably hydrochloric acid.

The cyclization of a compound of formula **(V)** or a salt thereof, can be carried out for example in the presence of a compound of formula **(VI).**

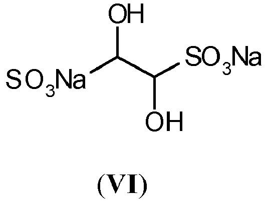

If desired, the reaction can be carried out in the presence of a solvent selected from an aprotic polar solvent, typically dimethylformamide, dimethylacetamide, acetonitrile, dimethylsulfoxide; a cyclic or acyclic ether, typically tetrahydrofuran, dioxane or methyl tert-butyl ether; a straight or branched C₁-C₆ alkanol, for example methanol, ethanol, isopropanol; a chlorinated solvent, typically dichloromethane; an apolar solvent typically toluene, an ester, for example ethyl acetate or a mixture of two or more, preferably two or three of said solvents, or in water or in an aqueous solution comprising one or more, preferably two or three of said solvents.

Preferably, the reaction is carried out in water or in an aqueous solution comprising one or more, preferably two or three of said solvents.

The compound **(II)** is known. As mentioned above, this can be obtained for example as disclosed in US 6,410,550. Anyway, such process makes use of osmium tetroxide dissolved in a solvent, for example acetone, or tert-butanol. As known, osmium is a toxic and very expensive heavy metal, which should then be removed from the product and disposed of as toxic industrial discarded. Therefore, its use is a limiting factor in the industrial scale production of a compound of formula **(II)** and therefore of Varenicline.

It has been surprisingly found a process for the preparation of a compound of formula **(II)** or a salt thereof, as defined above, comprising:
*cis*-dihydroxylating a compound of formula **(III)** 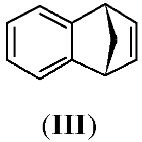
in the presence of an osmium compound, chosen from osmium tetroxide and potassium osmate dihydrate, supported on a resin to obtain a compound of formula **(VII)** 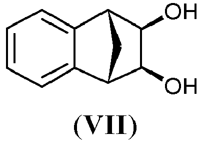
and subsequently converting it into a compound of formula **(II),** or a salt thereof.

The resin is typically a polymer supported resin. Such resin can be for example a hydrocarbon resin, preferably a polyethylene or polystyrene resin having pyridine or triethylamine residues.

In particular, osmium tetroxide is preferably supported on a polymer resin having pyridine residues while potassium osmate dihydrate is preferably supported on a polymer resin having triethylamine residues. Examples of these resins are FibreCat® 3003 and FibreCat® 3004, marketed by Johnson Matthey.

The *cis*-dihydroxylation reaction of a compound of formula **(III)** can be carried out in the presence of a co-oxidizing agent and, if necessary, of a solvent.

The co-oxidizing agent is preferably N-methyl morpholine N-oxide (NMO).

The osmium compound is typically used in catalytic amount, preferably in an amount comprised between 0.05% and 1% molar of an osmium compound to the alkene of formula **(III),** whereas the co-oxidizing agent is used in an at least stoichiometric amount.

The solvent is for example chosen from an aprotic polar solvent, typically dimethylformamide, dimethylacetamide, acetonitrile, dimethylsulphoxide; and ether, typically tetrahydrofuran or dioxane, and alcohol, for example methanol, ethanol, tert-butanol; a chlorinated solvent, typically dichloromethane; an apolar solvent, typically toluene, an ester, for example ethyl acetate; or water, or a mixture of two or more, preferably two or three of said solvents. Preferably the reaction is carried out in an aqueous mixture of tetrahydrofuran or tert-butanol.

The cis-dihydroxylation reaction can be carried out in a temperature range comprised between about 0°C and the reflux temperature of the reaction mixture. When the reaction is carried out in a tert-butanol aqueous mixture, the temperature of the reaction is preferably between about 50 and 70°C.

At the end of the reaction the resin can be removed from the solution containing the product of formula **(VII)** according to known techniques, for example by filtration. The so recovered resin can be used without further treatment in a subsequent reaction.

The conversion of a compound of formula **(VII)** to a compound of formula **(II)** or a salt thereof can be carried out according to what is disclosed in US 6,410,550.

The nitration reaction of a compound of formula **(II)** as defined above, no matter how it is carried out, provides the desired 1,2-dinitro derivative, obtained an major compound, and of its 1,3-regioisomer, obtained as minor product.

Thus for example the nitration of a compound of formula **(II),** according to the process of the invention, provides a mixture of a compound of formula **(IV)** or a salt thereof, as defined above, and formula **(IVb)** or a salt thereof 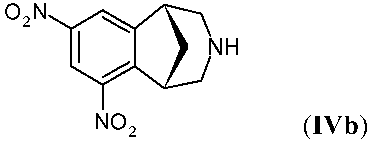 in a ratio of one another of about 70:30.

The separation on industrial scale of such isomers is rather complicated and slows down the industrial process.

It is herein found a process easily feasible on industrial scale which allows to separate a compound of formula **(IV)** from a mixture of the two compounds **(IV)** and **(IVb),** as previously defined.

Such process for the purification of a compound of formula **(IV),** as herein defined, which is a further object of the invention, comprises the separation of a compound of formula **(IV)** from a mixture of two compound of formula **(IV)** and **(IVb),** as defined above, by crystallization of a compound of formula **(IV)** from a solvent chosen from a straight or branched C₁-C₆ alkanol, preferably methanol or isopropanol.

In particular the so obtained compound of formula **(IV)** has a purity equal to or greater than 95%, being the regioisomer of formula **(IVb)** present as impurity in an amount lower than 0.1 %, measured by HPLC.

It is herein also found a process which allows the recovery of a compound of formula **(IVb),** which otherwise should be discarded with the effluent wastes of the process, by its conversion to the key compound of formula **(II),** or a salt thereof. Such operation renders the process of the present invention for the preparation of Varenicline more and more advantageous.

It is therefore a further object of the invention a process for the preparation of a compound of formula **(II)** or a salt thereof comprising:
- protecting the amino group in a compound of formula **(IVb)** or a salt thereof, 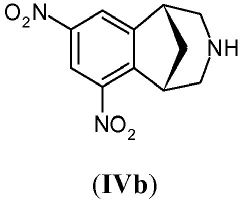 to obtain a compound of formula **(VIII),** wherein P is an amino protecting group, 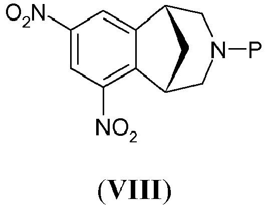
- reducing the nitro groups of a compound of formula **(VIII),** to obtain a compound of formula **(IX),** wherein P is as defined before, 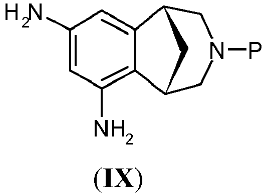
- deaminating a compound of formula **(IX),** to obtain a compound of formula **(X)** 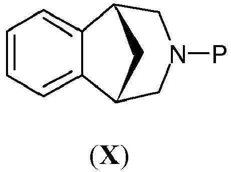 wherein P is as defined above; and
- removing the protective group in a compound of formula **(X)** and if desired, converting a so obtained compound of formula **(II),** to a salt thereof.

The protection of the secondary amino group and its subsequent removal can be carried out according to known techniques.

The amino protecting group P can be a group commonly used for protecting the secondary amines, for example benzyl, a COOR where R is allyl, a C₁-C₆ alkyl group or aryl-C₁-C₉ alkyl group; a CONR¹R² group wherein R¹ and R² can be independently chosen from hydrogen and C₁-C₆ alkyl, or R¹ and R², taken together at the nitrogen atom to which they are bound, form a pyrrolidine, piperidine or morpholine ring; a CHO group; or CO(C₁-C₆)alkyl group.

In such protecting group a C₁-C₆ alkyl group or C₁-C₉ alkyl group can be straight or branched and optionally substituted with one or more halogen atoms, preferably from one to three fluorine or chlorine atoms; preferably P is acetyl or trifluoroacetyl.

The reduction of the nitro groups in a compound of formula **(VIII)** to obtain a compound of formula **(IX)** can be carried out as previously disclosed for the conversion of a compound of formula **(IV)** to a compound of formula **(V).**

A compound of formula **(IX)** can then be submitted to deamination to obtain a compound of formula **(X)** for example via diazonium salts, as known in the art.

In the context of the invention a salt of a compound of formula **(I), (II), (IV), (IVb), (V)** and **(IX),** as herein defined, is typically a pharmaceutically acceptable salt thereof for example the hydrochloride, fumarate, tartrate, citrate, succinate, tosylate.

A compound of formula **(I),** and also a compound of formula **(II), (IV), (IVb), (V)** or **(IX),** as herein defined, can be converted to a salt thereof, or vice versa, according to known methods.

The following examples illustrate the invention.

### Example 1: Preparation of a compound of formula (VII)

A solution composed of 200 mL of tert-butanol and 60 mL of water is loaded into a four-necked 500 mL round flask fitted with magnetic stirrer, reflux condenser, thermometer and rendered inert in nitrogen atmosphere.

A compound of formula **(III)** (25.43 g, 0.179 mols) and N-methylmorpholine N-oxide (27.83 g, 0. 79 mols) are added. The mixture is left under stirring till the complete dissolution of the solids and then FibreCat® 3003 (0.730 g; 0.1% molar in osmium) is added. The reaction mixture is heated at 60°C and maintained under stirring till the quantitative conversion of the starting compound **(III)** into the diol **(VII).**

At the end of the reaction the resin is filtered off, and the hydroalcoholic solution, which contains the reaction product is concentrated under reduces pressure. The residue is diluted with acetone and the obtained suspension is heated at 55°C for one hour. The suspension is cooled and the solid filtered on Buckner and dried at a temperature of 45°C for 2 hours. 19.5 g of the intermediate **(VII)** are obtained with a yield of 70%.

### Example 2: Preparation of the hydrochloride salt of a compound of formula (II)

The compound of formula **(VII)** (31 g, 0,175 mol) and triethylammoniumbenzyl chloride (8.0 g, 0.035 mols) are dissolved in dichloromethane (930 mL) in a five-necked 3 L round flask endowed with magnetic stirrer, reflux condenser, thermometer, dropping funnel and rendered inert in nitrogen atmosphere.

659.4 g of an aqueous solution of sodium metaperiodate (5.97%, 0.184 mols) are added to the mixture in about 10 minutes and maintaining the temperature of the mixture below 30°C. The reaction mixture is maintained under strong stirring for 1 hour and 30 minutes. The phases are separated and the organic one is first submitted to repeated aqueous washings till the test with amidoiodinated paper of the washing waters results to be negative. Then it is dried with Na₂SO₄. Then, benzylamine (19.4 g, 0.181 mols) and triethylammoniumbenzylchloride (1.0 g, 4.0 mmols) are added into the anhydrous methylene solution. The solution is dripped in 30 minutes in a suspension of sodium triacetoxyborohydride (115 g, 0.54 mols) in dichloromethane (620 mL) cooled in an ice bath.

The suspension is left under stirring for two hours and the temperature of the mixture is left to slowly go up to 25°C. The reaction mixture is quenched in an aqueous solution of 25% K₂CO₃. The phases are separated and the methylene solution is washed with water, dried on Na₂SO₄ and concentrated at reduced temperature. 43.74 g of an oily residue are obtained. The so obtained residue (43.74 g, 0,175 mols) is dissolved in 450 mL of methanol and 44.4 g of Pd/carbon at 5% w/w (containing 58% w/w of water) are added to the solution. The environment reaction is saturated with hydrogen at 1 atm and left to react for 18h at 20°C. The carbon is filtered off on a celite panel and then methanol is distilled under reduced pressure. The so obtained oily residue is diluted in dichloromethane and the organic solution is washed with water.

The organic phase is dried on Na₂SO₄, filtered and concentrated under reduced pressure. The obtained oil is dissolved in 110 mL of acetone and the solution is added, with no heating, with gaseous hydrochloric acid till a pH comprised between 2 and 3. After one hour the hydrochloride salt of the intermediate of formula **(II)** (23.19 g, 0.12 mols) is filtered off and obtained with a yield of 68% in three steps starting from the compound of formula **(VII).**

### Example 3: Preparation of a compound of formula (IV)

Sulphuric acid (11.14 g at 98% w/w, about 6mL) is loaded into a 50 mL flask, rendered inert under nitrogen atmosphere. The acid is cooled to 0°C, and then fuming nitric acid (13 g, 0.204 mols) is slowly added and the so obtained solution is maintained under stirring at 0°C for about one hour. The hydrochloride salt of the compound of formula **(II)** (4.0 g, 20.4 mmols) is then added as a solid, in parts. After the addition of the substrate the reaction mixture is slowly heated at about 50°C and left under stirring at the same temperature for further 4 hours. The reaction mixture is then quenched in an aqueous solution of 20% NaCl cooled to a temperature comprised between -5°C and 0°C, and the obtained mixture is maintained under stirring for 30 minutes. The obtained solid is filtered off and further washed with an aqueous solution of 10% NaCl. The recovered solid is dried in oven at a temperature of 60°C. 5.46 g of a mixture composed of the hydrochloride salts of the compounds of formula **(IV)** and **(IVb)** are obtained, in a molar ratio one another between about 73:27, both evaluated by HPLC and by ¹H-NMR.

The so obtained mixture of the two regioisomers is thus suspended in water, cooled to 0°C and treated with an aqueous solution of 30% NaOH. The mixture is maintained under stirring for about 30', then extracted more times with dichloromethane. The organic phase is then dried with sodium sulphate, filtered off and the solvent is evaporated under reduced pressure.

The obtained residue is then suspended in methanol and the suspension is heated at reflux of the solvent. The solution under stirring is then left to slowly cool till a temperature of 25°C, and maintained at the same temperature for further 3 hours. The solid is filtered off and dried in oven at a temperature of 50°C. 2.9 g of the compound of formula **(IV)** are obtained, with a purity greater than 95%, a content of the regioisomer **(IVb)** lower than 0.1 % and a yield of 57%.

### Example 4: Preparation of compound Varenicline (I)

A solution prepared dissolving a compound of formula **(IV)** (30.0 g, 0.12 mols) in 440 mL of tetrahydrofuran is loaded in a 1L flask.

33 g of Pd/C (containing 58.5% w/w of water, 3.97 mmols of Pd, 3% molar) are added, maintaining the reaction mixture under stirring. The reaction environment is saturated with hydrogen at atmospheric pressure and left to react for 48 h at room temperature. The suspension is filtered on celite and the panel is first washed with tetrahydrofuran and then with water. The collected organic phases are directly transferred in a 2L flask, whereas the aqueous phase is first treated with the glyoxal sodium bisulphite adduct **(VI)** (34.3 g, 0.13 mols) and then added to the organic phase. The so obtained biphasic mixture is heated at 55°C and maintained under strong stirring for two hours. The phases are separated and the organic one is concentrated under reduced pressure. The obtained residue is added to the aqueous phase previously removed. The aqueous mixture is treated with a solution of 30% NaOH till it is actually basic. The product is extracted more times with dichloromethane and the organic phases are collected and dried with anhydrous sodium sulphate, filtered off and the solvent is removed by distillation under reduced pressure. Solid Varenicline (I) (20.5 g) is obtained with a yield of 70% in two steps starting from the compound of formula **(IV).**

## Claims

1. Process for the preparation of a compound of formula **(I)** or a salt thereof, comprising: 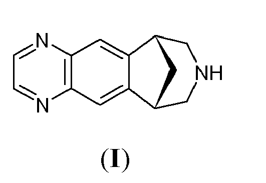 nitrating a compound of formula **(II)** or a salt thereof, 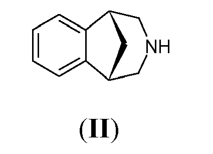 to obtain a compound of formula **(IV)** or a salt thereof, 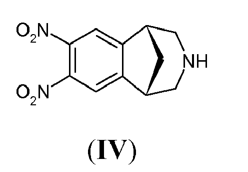 reducing it, to obtain a compound of formula **(V)** or a salt thereof, and
then 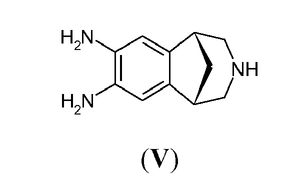 cyclizing it, to obtain a compound of formula **(I)** or a salt thereof, and, if desired, converting a compound of formula **(I)** to a salt thereof, or the salt of a compound of formula **(I)** to the free compound, **characterized in that**:
the nitration of a compound of formula **(II)** or a salt thereof, is carried out with concentrated nitric acid in presence of a strong inorganic acid, and the amino group in the compound of formula **(II)** is not protected.

2. Process according to claim 1, wherein the concentrated nitric acid is fuming nitric acid.

3. Process according to claim 1 wherein the strong inorganic acid is chosen from sulphuric acid, polyphosphoric acid and perchloric acid, preferably sulphuric acid.

4. Process according to claim 1 further comprising preparing a compound of formula **(II)** or a salt thereof, as defined in claim 1, by a process comprising:
*cis*-dihydroxylating a compound of formula **(III)** 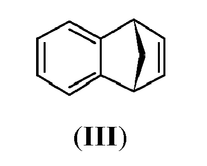
in presence of an osmium compound chosen from osmium tetroxide and potassium osmate dihydrate on a resin support, to obtain a compound of formula **(VII)** 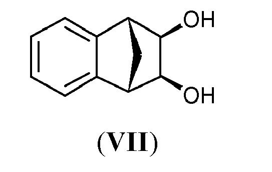
and then converting it to a compound of formula **(II),** or a salt thereof.

5. Process according to claim 4, wherein the resin is a substituted polymer resin, preferably a hydrocarbon resin, substituted by pyridine or triethylamine residues.

6. Process according to claims 4 and 5 wherein osmium tetroxide is supported on a polymer resin substituted by pyridine residues; and potassium osmate dihydrate is supported on a polymer resin substituted by triethylamine residues.

7. Process according to claim 4 wherein the *cis*-dihydroxylation of a compound of formula **(III)** is carried out in presence of a co-oxidizing agent preferably N-methylmorpholine N-oxide (NMO), and, if necessary, of a solvent.

8. Process according to claim 4 wherein the osmium compound is used in catalytic amount, preferably in a molar ratio of the osmium compound to the compound of formula **(III)** from 0.05% to 1% molar, and the co-oxidizing agent is used in stoichiometric ratio.

9. Process according to claim 1 further comprising the purification of a compound of formula **(IV),** from a mixture of compounds of formula **(IV)** and **(IVb),** 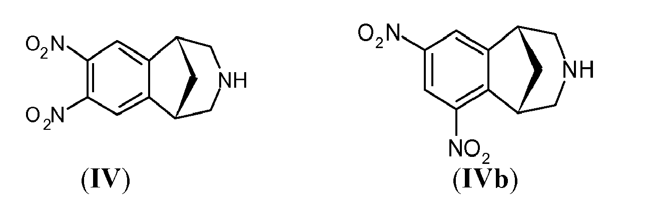 by crystallizing from a solvent chosen from a straight or branched C₁-C₆ alkanol, preferably methanol or isopropanol.

10. Process according to claim 1 and 9 further comprising recovering a compound of formula **(IVb)** by converting it into a compound of formula **(II),** or a salt thereof, comprising:
- protecting the amino group in a compound of formula **(IVb)** or a salt thereof 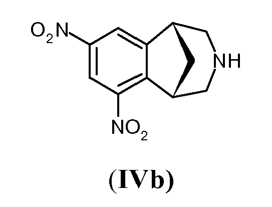 to obtain a compound of formula **(VIII),** wherein P is an amino protecting group 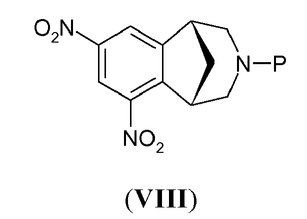
- reducing the nitro groups in a compound of formula **(VIII),** to obtain a compound of formula **(IX),** wherein P is as defined above, 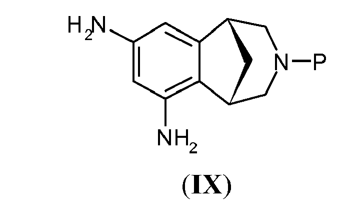
- deaminating a compound of formula **(IX),** to obtain a compound of formula **(X),** 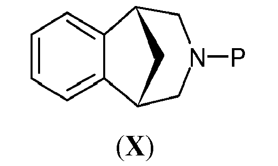 wherein P is as defined above and
- removing the protecting group in a compound of formula **(X),** and, if desired, converting the so obtained compound of formula **(II)** to a salt thereof.
